# EUROPÄISCHE PATENTSCHRIFT

(11) **EP 1 195 170 B1**
(45) Veröffentlichungstag und Bekanntmachung des Hinweises auf die Patenterteilung: **17.12.2003**
(21) Anmeldenummer: 00121825.4
(22) Anmeldetag: 06.10.2000
(51) Int. Cl.: A61M 1/00

(54) **Ventil für eine Muttermilchpumpe und Muttermilchpumpe**
Valve for a breastpump and breastpump
Valve pour un tire-lait et tire-lait

(43) Veröffentlichungstag der Anmeldung: 10.04.2002
(73) Patentinhaber: Nüesch-Engineering, 5453 Remetschwil (CH)
(72) Erfinder: Nüesch, Hansueli, 5453 Remetschwil (CH)
(74) Vertreter: Stocker, Kurt

(56) Entgegenhaltungen:
- EP-A- 0 194 076
- DE-A- 19 747 842
- GB-A- 1 233 235

## Beschreibung

Die Erfindung bezieht sich auf ein Ventil für eine Muttermilchpumpe und eine Muttermilchpumpe mit den Merkmalen des Oberbegriffs des Anspruchs 5 bzw. des Anspruches 1. Ein derartiges Ventil ist aus der DE-A-197 47 842 bekannt geworden. Dabei ist ein Gehäuse mit einer Einlaßöffnung an einem Ende und einer Ventilöffnung am anderen Ende vorgesehen. Der Einlaßöffnung gegenüber liegt eine Stirnfläche eines länglichen Ventilkörpers für den Überlaufschutz, so daß im Falle von in das Gehäuse eintretender Milch erst diese Fläche von ihr beaufschlagt wird und somit der Ventilkörper die gegenüberliegende Ventilöffnung verschließt, bevor die Milch Zeit genug hat, am Ventilkörper vorbei und in den Bereich der Ventilöffnung zu gelangen. Der Ventilkörper ist dabei an einer Trägermembrane aufgehängt, die mit einander gegenüberliegenden oder sternförmig um den Ventilkörper herum angeordneten Armen den Ventilkörper zu einer geradlinigen Bewegung gegen die Ventilöffnung hin führt.

In der Praxis hat sich diese Anordnung recht gut bewährt, denn das Eindringen von Milch in die Pumpeneinheit würde diese so stark schädigen, daß sie ausgewechselt werden müßte. In der Praxis hat sich aber gezeigt, daß der Ventilkörper, und damit auch sein Gehäuse, zweiteilig und doch relativ lang ausgebildet werden muß. Dies verteuert die Herstellung des Ventils und macht es dazu verhältnismäßig platzaufwendig.

Der Erfindung liegt nun die Aufgabe zugrunde, ein Ventil der eingangs genannten Art so auszubilden, daß ein sicherer Verschluß auch bei einer kleineren Bauweise möglich ist. Dies gelingt erfindungsgemäß durch die kennzeichnenden Merkmale des Anspruches 5.

Einerseits wird so ein vorher nicht ohne weiteres zu erkennender strömungsdynamischer Effekt geschaffen: Eintretende Milch spritzt durch die Einlaßöffnung gegen die obere Stirnfläche des hohlen Ventilkörpers und bringt diesen in die Schließstellung. Auch wenn der Ventilkörper, im Vergleich mit dem Stand der Technik, relativ kurz ist, so ist doch der Weg der Milch deshalb relativ lang, weil sie von der oberen Stirnfläche des Hohlkörpers nach unten abgelenkt wird und dann wieder aufwärts steigen muß, um zur Ventilöffnung gelangen zu können. Das bedeutet, daß der Weg der Milch von innen nach außen gefaltet wird. Zudem wird vom unten offenen, hohlen Ventilkörper nur ein geringes Volumen beansprucht, sodass im Bereich des Ventilkörpers relativ viel Milch aufgenommen werden kann, bis dieser Bereich bis zum Ventil mit Milch gefüllt ist.

Daneben hat sich aber auch noch ein anderer günstiger Effekt gezeigt: Dadurch, daß der Ventilkörper hohl ist und seine untere Stirnwand, die bisher für den Schließeffekt bei Beaufschlagung durch Milch bestimmend war, wegfällt, ferner aber auch der ganze Ventilkörper kürzer wird, ergibt sich eine geringere Masse. Sieht man aber die Einheit aus, vorzugsweise membranartigem, Träger und Ventilkörper als ein schwingungsfähiges Masse-Feder-System an, so wird klar, daß mit der Verringerung der Masse die Eigenfrequenz des Systems steigt, d.h. daß das erfindungsgemäße Ventil noch schneller schließt als nach dem Stand der Technik und deshalb auch noch kleiner gebaut werden kann.

Dazu ergibt sich ein weiterer Effekt dadurch, daß die bisherige Geradführung durch mehrere Arme des Trägers zwangsläufig eine gewisse Steifheit mit sich brachte. Dadurch, daß nun die Anlenkung einseitig ist (wobei der Träger vorzugsweise von einem flexiblen Körper, insbesondere in bekannter Weise von einer Membran gebildet wird, aber gegebenenfalls auch ein fester Körper mit einem Scharniergelenk sein könnte), steigert sich die Beweglichkeit und der obige Effekt der Beschleunigung des Ansprechens wird noch deutlicher.

Obwohl die Öffnung des nach unten offenen Hohlkörpers beliebige Dimensionen haben könnte, ergibt sich die erwähnte günstige Strömungsdynamik besonders dann, wenn die Öffnung den vorbestimmten Querschnitt hat und so mit der Mantelfläche des Hohlraumes fluchtet.

Eine weitere Beschleunigung und Sicherung der Funktion ergibt sich dadurch, daß wenigstens ein Teil der aus Ventilkörper und Träger bestehenden Einheit aus einem Material mit einer geringeren Dichte als 1,034 g/c, vorzugsweise unter 1,028 g/c, besteht. Dem liegt der Gedanke zugrunde, daß mit einer solchen Materialwahl auch ein gewisser Schwimmeffekt genutzt wird, weil Muttermilch in der Regel eine Dichte von etwa 1,034 g/c haben wird, in selteneren Fällen von 1,028 g/c. Somit ist ein starker Auftrieb schon dann gesichert, wenn eindringende Milch die genannte Einheit erreicht.

Ein besonders günstiges Material zur Erzielung dieses Effektes ist Silikongummi, weil dieses Material auch lebensmitteltauglich und im Bedarfsfall leicht zu reinigen ist.

Ein erfindungsgemäßes Ventil weist demnach die Merkmale des Anspruches 5 auf.

Weitere Einzelheiten der Erfindung ergeben sich an Hand der nachfolgenden Beschreibung eines in der Zeichnung schematisch dargestellten, besonders bevorzugten Ausführungsbeispieles. Es zeigen:
- Fig. 1: einen Axialschnitt durch eine Einheit aus Brustkörper, Sauganschluß und dazwischenliegendem erfindungsgemäßen Ventil;
- Fig. 2: eine gegenüber Fig. 1 vergrößerte Darstellung, welche nur das erfindungsgemäße Ventil am Sauganschluß zeigt; und die
- Fig. 3 und 4: die Einheit von Trägermembrane und hohlem Ventilkörper in je einer Perspektivansicht von oben und unten.

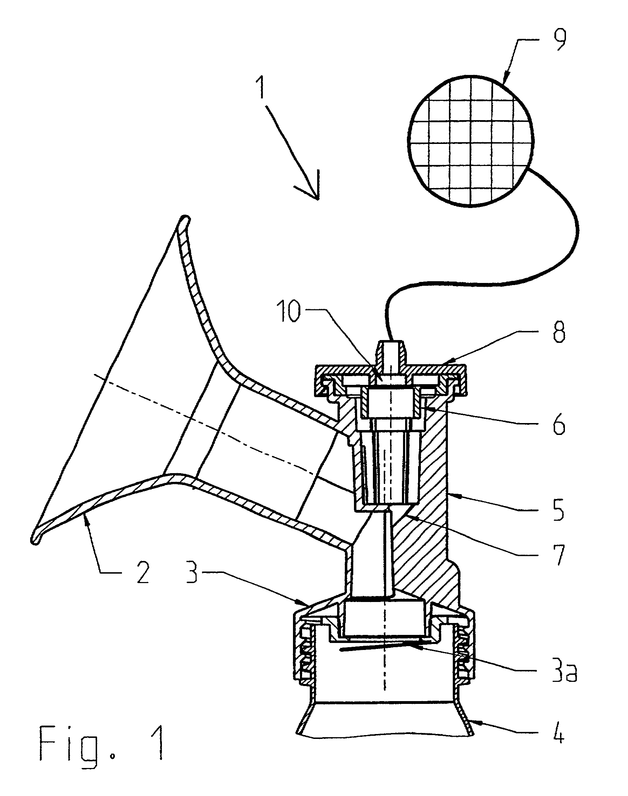

Die Fig. 1 zeigt eine Muttermilchpumpe 1 mit einem Brustkörper 2, einem Verbindungsbereich 3 zu einem an diesem festschraubbaren Sammelbehälter 4 und einem Ventilgehäuse 5, in dem ein Ventilkörper 6 bewegbar angeordnet ist. Dem Verbindungsbereich 3 ist vorzugsweise ein Rückschlagventil 3a zugeordnet, welches während der Saugphasen schliesst und durch welches Muttermilch vom Brustkörper 2 in den Sammelbehälter 4 strömen kann. Das Innere des Ventilgehäuses 5 ist über eine Einlassöffnung 7 an einer ersten Stirnseite des Ventilgehäuses 5 mit dem Brustkörper 2 und über einen Sauganschluss 8 mit einer Saugpumpe 9 verbunden. Um zu verhindern, dass Milch in die Saugpumpe 9 gesaugt wird, ist der Ventilkörper 6 von Milch, welche durch die Einlassöffnung 7 ins Ventilgehäuse 5 eintritt, gegen eine Verbindungsöffnung 10 des Sauganschlusses 8 bewegbar.

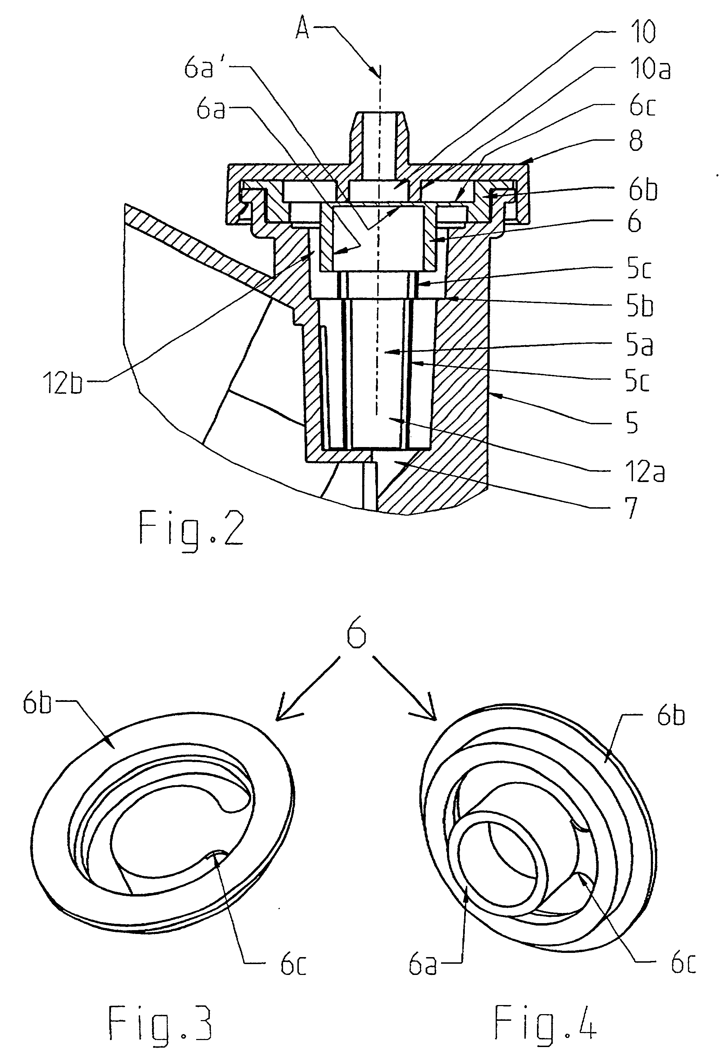

Gemäss Fig. 2 hat das Ventilgehäuse 5 einen Innenraum 5a, der sich entlang einer Achse A von der Einlassöffnung 7 bis zur Verbindungsöffnung 10 erstreckt. In der dargestellten Ausführungsform erweitert sich der Querschnitt des Innenraumes 5a von der Einlassöffnung 7 gegen die Verbindungsöffnung 10 hin. Die Erweiterung umfasst vorzugsweise eine Erweiterungsstufe 5b, die zwischen der Einlassöffnung und dem Ventilkörper 6 ausgebildet ist. Dadurch können die Aussenmasse des Ventilkörpers 6 im wesentlichen entsprechend dem kleineren Querschnitt bei der Erweiterungsstufe 5b gewählt werden, ohne dass sich der Ventilkörper 6 im Innenraum verklemmen kann. Wenn nun schwallartig Milch in den Innenraum 5a eintritt, trifft die Milch auf den Ventilkörper 6. Ein direktes Treffen des Ringraumes um den Ventilkörper 6 ist aufgrund der Erweiterungsstufe sehr unwahrscheinlich.

Der Ventilkörper 6 umfasst einen zylindrischen Ventilteil mit einer Mantelfläche 6a und einer Abschlussfläche 6a', einen Träger 6b und einen Verbindungsbereich 6c. Der zylindrische Ventilteil bildet einen Hohlkörper mit einer, der Einlaßöffnung 7 zugekehrten, Öffnung. Die Abschlussfläche 6a' ist der Verbindungsöffnung 10 zugeordnet und der zylindrische Ventilteil ist mit dem Träger 6b über eine bewegliche Verbindung schwenkbar verbunden. Ausgehend von der Einlassöffnung 7 führt ein erster Verbindungskanal-Abschnitt 12a zum Ventilkörper 6. Im Bereich des Ventilkörpers 6 ist als zweiter Verbindungskanal-Abschnitt ein in Achsrichtung verlaufender Ringraum 12b zwischen dem Ventilkörper 6 und dem Ventilgehäuse 5 ausgebildet. Um auch bei schräg stehendem zylindrischem Ventilteil einen problemlosen Durchtritt von Luft zu gewährleisten, sind an der Innenwand des Ventilgehäuses 5 gegebenenfalls Längsrippen 5c ausgebildet.

Gemäss Fig. 3 und 4 ist der bevorzugte Ventilkörper 6 einteilig ausgebildet. Der Träger 6b ist ringförmig und passt in einen Aufnahmebereich zwischen dem Ventilgehäuse 5 und dem Sauganschluss 8. Durch das Aufsetzen des Sauganschlusses 8 auf das Ventilgehäuse 5 wird der Träger 6b festgeklemmt. Die Mantelfläche 6a und die Abschlussfläche 6a' sind über den Verbindungsbereich 6c mit dem Träger 6b verbunden, wobei zumindest der Verbindungsbereich 6c aus elastischem Material gebildet ist und so eine Schwenkbewegung gewährleistet wird. Wenigstens ein Teil der aus Ventilkörper 6 und Träger 6b bestehenden Einheit besteht aus einem Material mit einer geringeren Dichte als 1,034g/c, vorzugsweise unter 1,028g/c. Dem liegt der Gedanke zugrunde, daß mit einer solchen Materialwahl auch ein gewisser Schwimmeffekt genutzt wird, weil Muttermilch in der Regel eine Dichte von etwa 1,034 g/cm³ haben wird, in selteneren Fällen von 1,028 g/cm³. Um die Verbindungsöffnung 10 bei einer Bewegung des Ventilkörpers 6 gegen die Verbindungsöffnung 10 verschliessbar zu machen, steht ein Ringbereich 10a von der Berandung der Verbindungsöffnung 10 in Achsrichtung bis zur Ebene mit der nicht ausgelenkten Abschlussfläche 6a', bzw. mit dem Verbindungsbereich 6c, vor. Beim Abpumpen von Milch ist das Ventilgehäuse 5 so ausgerichtet, dass die Mantelfläche 6a mit der Abschlussfläche 6a' aufgrund des Eigengewichtes etwas nach unten ausgelenkt wird, wobei der Verbindungsbereich 6c etwas durchgebogen ist. Die Auslenkung führt dazu, dass eine Saugverbindung von der Verbindungsöffnung 10 zur Einlassöffnung 7 offen, bzw. durchgängig ist.

Wenn nun während einer Saugphase der über den Sammelbehälter angestiegene Milchspiegel die Einlassöffnung 7 erreicht, so gelangt ein Milchstrahl in das Innere des Ventilteiles mit der Mantelfläche 6a und der Abschlussfläche 6a'. Durch die Strahlumlenkung an der Abschlussfläche 6a' erhält das Ventilteil 6a, 6a' einen Druck- bzw. Kraftstoss, der genügt um die Abschlussfläche 6a' an den Ringbereich 10a zu bewegen, wo die Abschlussfläche 6a' während der Saugphase haften bleibt und somit ein Ansaugen von Milch durch die Verbindungsöffnung 10 verhindert. Falls der Druckstoss und der Ansaugeffekt nicht genügen, um die Verbindungsöffnung 10 zu verschliessen, so führt die im Verbindungskanal 12a, 12b befindliche Milch bei weiteren Saugzyklen zu einem erhöhten Umströmungswiderstand und zu einer auf den Ventilkörper wirkenden Auftriebskraft, welche beiden Effekte eine zum Verschliessen der Verbindungsöffnung 10 benötigte Bewegung des Ventilteiles 6a, 6a' bewirken. Wenn die Milch im Ventilgehäuse 5 bis zur Mantelfläche 6a aufsteigen sollte, so erzielt der teilweise mit Luft gefüllte, von der Mantelfläche 6a umgebene Hohlraum im Ventilteil einen grossen Auftrieb und ein Anpressen der Abschtussffäche 6a' an den Öffnungs-Ringbereich 10a. Weil die Unterseite der Abschlussfläche 6a' und nicht eine Fläche bei der Einlaßöffnung 7 als Prallfläche für den eintretenden Milchschwall eingesetzt wird, kann im Ventilgehäuse 5, ein grosses Volumen bereitgestellt werden, das Milch aufnimmt, bevor die Milch bis zur Verbindungsöffnung 10 gelangen könnte. Zudem kann der erfingungsgemässe Ventilkörper 6 mit einem kurzen Mantel äusserst leicht ausgebildet werden, so dass bereits ein kleiner Milchstahl den zum Schliessen nötigen Kraftstoss erzielt. Die Erweiterungsstufe 5b und die optimale Wahl des Querschnittes des Ventilkörpers 5 reduzieren die Gefahr eines direkten Milch-Strahlenweges von der Einlassöffnung 7 durch den Verbindungskanal 12a,12b zur Verbindungsöffnung 10. Gegebenenfalls wird die Erweiterungsstufe 5b so stark ausgebildet, dass der Querschnitt des Innenraumes 5a kleiner ist als der Querschnitt der Mantelfläche 6a. Der Ventilkörper 6 ist klein und vorzugsweise einteilig gebaut und gewährleistet einen sicheren Verschluß.

Bei Muttermilchpumpen mit zwei Brustkörpern wird vorzugsweise jedem Brustkörper ein Überlaufschutz mit einer Ventilanordnung zugeordnet. Gegebenenfalls aber genügt ein Überlaufschutz der an einen gemeinsamen Verbindungsbereich der beiden Brustkörper anschliesst.

Der für eine Muttermilchpumpe entwickelte Überlaufschutz mit dem Ventilgehäuse 5 und dem darin bewegbaren Ventilkörper 6 ist auch in anderen medizinischen Absauggeräten vorteilhaft einsetzbar.

### Bezugszeichenliste

- 1: Muttermilchpumpe
- 2: Brustkörper
- 3: Verbindungsbereich zw. 2 + 4
- 3a: Rückschlagventil
- 4: Sammelbehälter
- 5: Ventilgehäuse
- 5a: Innenraum
- 5b: Erweiterungsstufe
- 5c: Längsrippen
- 6: Ventilkörper
- 6a: Mantelfläche
- 6a': Abschlussfläche
- 6b: Träger
- 6c: Verbindungsbereich
- 7: Einlassöffnung
- 8: Sauganschluß
- 9: Saugpumpe
- 10: Verbindungsöffnung
- 10a: Ringbereich
- 12a: erster Verbindungskanal-Abschnitt
- 12b: Ringraum

## Patentansprüche

1. Muttermilchpumpe (1) mit zumindest einem Brustkörper (2), einem mit einer Saugpumpe (9) verbindbaren Sauganschluß (8), einer Sperreinrichtung für Milch, die einen eine Mantelfläche (6a) besitzenden Ventilkörper (6) aufweist, der axial beweglich mittels eines Trägers (6b) an einer Verbindungsöffnung (10) in einem Ventilgehäuse (5) gehalten ist, welches mit einer dem Ventilkörper (6) etwa gegenüberliegenden Einlaßöffnung (7) versehen ist, zwischen dem mindestens einen Brustkörper (2) und dem Sauganschluß (8) und einem an den mindestens einen Brustkörper (2) anschließenden Verbindungsbereich (3) zum Ableiten der Milch in einen Sammelbehälter (4), **dadurch gekennzeichnet, daß** der Ventilkörper (6) als Hohlkörper mit einer der Einlaßöffnung (7) zugekehrten Öffnung zu seinem von der Mantelfläche (6a) begrenzten Hohlraum vorbestimmten Querschnittes ausgebildet ist, und daß dieser Ventilkörper (6) an seinem Träger (6b) um eine, allenfalls gedachte, Gelenkachse einseitig frei schwenkbar ist.

2. Muttermilchpumpe nach Anspruch 1, **dadurch gekennzeichnet, daß** die Öffnung den vorbestimmten Querschnitt hat und so mit der Mantelfläche (6a) des Hohlraumes fluchtet.

3. Muttermilchpumpe nach Anspruch 1 oder 2, **dadurch gekennzeichnet, daß** wenigstens ein Teil der aus Ventilkörper (6) und Träger (6b) bestehenden Einheit aus einem Material mit einer geringeren Dichte als 1,034 g/cm³, vorzugsweise unter 1,028 g/cm³, besteht.

4. Muttermilchpumpe nach Anspruch 3, **dadurch gekennzeichnet, daß** wenigstens ein Teil der aus Ventilkörper (6) und Träger (6b) bestehenden Einheit, insbesondere der Träger (6b), aus Silikongummi besteht.

5. Ventil für eine Muttermilchpumpe nach einem der vorhergehenden Ansprüche, mit einem eine Mantelfläche (6a) besitzenden Ventilkörper (6), der axial beweglich mittels eines Trägers (6b) an einer Verbindungsöffnung in einem Ventilgehäuse (5) gehalten ist, welches andernends mit einer dem Ventilkörper (6) etwa gegenüberliegenden Einlaßöffnung (7) versehen ist, **dadurch gekennzeichnet, daß** der Ventilkörper (6) als Hohlkörper mit einer der Einlaßöffnung (7) zugekehrten Öffnung zu seinem von der Mantelfläche (6a) begrenzten Hohlraum vorbestimmten Querschnittes ausgebildet ist, und daß dieser Ventilkörper (6) an seinem Träger (6b) um eine, allenfalls gedachte, Gelenkachse einseitig frei schwenkbar ist.

6. Ventil nach Anspruch 5, **dadurch gekennzeichnet, daß** die Öffnung den vorbestimmten Querschnitt hat und so mit der Mantelfläche (6a) des Hohlraumes (5a) fluchtet.

7. Ventil nach Anspruch 5 oder 6, **dadurch gekennzeichnet, daß** wenigstens ein Teil der aus Ventilkörper (6) und Träger (6b) bestehenden Einheit aus einem Material mit einer geringeren Dichte als 1,034 g/cm, vorzugsweise unter 1,028 g/cm, besteht.

8. Ventil nach Anspruch 7, **dadurch gekennzeichnet, daß** wenigstens ein Teil der aus Ventilkörper (6) und Träger (6b) bestehenden Einheit, insbesondere der Träger (6b), aus Silikongummi besteht.

## Claims

1. Breast pump (1) with at least one breast body (2), a suction connection (8) which can be connected to a suction pump (9) and a blocking device for milk, comprising a valve body (6) having a circumferential surface (6a) and mounted in an axially movable manner by means of a support (6b) on a connection opening (10) in a valve housing (5) provided with an inlet opening (7) situated substantially opposite the valve body (6) between the at least one breast body (2) and the suction connection (8) and a connecting region (3) adjoining the at least one breast body (2) for discharging the milk into a collecting container (4), **characterised in that** the valve body (6) is designed as a hollow body with an opening directed towards the inlet opening (7) leading to its hollow space having a predetermined cross section delimited by the circumferential surface (6a) and that this valve body (6) is mounted in such a manner on its support (6b) that it can swivel freely at one side about an, if necessary, imaginary hinge axis.

2. Breast pump according to claim 1, **characterised in that** the opening has the predetermined cross section and is therefore aligned with the circumferential surface (6a) of the hollow space.

3. Breast pump according to claim 1 or claim 2, **characterised in that** at least part of the unit consisting of the valve body (6) and the support (6b) consists of a material having a density of less than 1.034 g/cm, preferably less than 1.028 g/cm.

4. Breast pump according to claim 3, **characterised in that** at least part of the unit consisting of the valve body (6) and the support (6b), in particular the support (6b), consists of silicone rubber.

5. Valve for a breast pump according to one of the preceding claims, comprising a valve body (6) having a circumferential surface (6a) and mounted in an axially movable manner by means of a support (6b) on a connection opening in a valve housing (5) provided at its other end with an inlet opening (7) situated substantially opposite the valve body (6), **characterised in that** the valve body (6) is designed as a hollow body with an opening directed towards the inlet opening (7) leading to its hollow space having a predetermined cross section delimited by the circumferential surface (6a) and that this valve body (6) is mounted on its support (6b) in such a manner that it can swivel freely at one side about an, if necessary, imaginary hinge axis.

6. Valve according to claim 5, **characterised in that** the opening has the predetermined cross section and is therefore aligned with the circumferential surface (6a) of the hollow space (5a).

7. Valve according to claim 5 or claim 6, **characterised in that** at least part of the unit consisting of the valve body (6) and the support (6b) consists of a material having a density of less than 1.034 g/cm, preferably less than 1.028 g/cm.

8. Valve according to claim 7, **characterised in that** at least part of the unit consisting of the valve body (6) and the support (6b), in particular the support (6b), consists of silicone rubber.

## Revendications

1. Tire-lait (1) comportant au moins un corps pour sein (2), un raccordement pour aspiration (8) susceptible d'être relié à une pompe d'aspiration (9), un dispositif de blocage pour le lait, présentant un opercule (6) comportant une surface d'enveloppe (6a), opercule maintenu de façon axialement mobile au moyen d'un support (6b) sur une ouverture de liaison (10) ménagée dans un boîtier de soupape (5), qui est muni d'une ouverture d'admission (7) située à peu près à l'opposée de l'opercule (6), entre le au moins un corps pour sein (2) et le raccordement par aspiration (8) et une zone de liaison (3), se raccordant au au moins un corps pour sein (2), pour évacuer le lait et l'amener à un récipient collecteur (4), **caractérisé en ce que** l'opercule (6) est réalisé sous la forme de corps creux avec une ouverture tournée vers l'ouverture d'admission (7), pour former son espace creux, délimité par la surface d'enveloppe (6a) et de section transversale prédéterminée, et **en ce que** cet opercule (6) est susceptible de pivoter librement sur une extrémité sur son support (6b), autour d'un axe d'articulation en tout cas imaginaire.

2. Tire-lait selon la revendication 1, **caractérisé en ce que** l'ouverture présente la section transversale prédéterminée et est ainsi en alignement avec la surface d'enveloppe (6a) de l'espace creux.

3. Tire-lait selon la revendication 1 ou 2, **caractérisé en ce qu'**au moins une partie de l'ensemble, formé de l'opercule (6) et du support (6b), est formée d'un matériau d'une masse volumique inférieure à 1,034 g/cm³, de préférence inférieure à 1,028 g/cm³.

4. Tire-lait selon la revendication 3, **caractérisé en ce qu'**au moins une partie de l'ensemble, formé de l'opercule (6) et du support (6b), en particulier le support (6b), est formée en caoutchouc au silicone.

5. Soupape formant clapet pour un tire-lait selon l'une des revendications précédentes, avec un opercule (6) présentant une surface d'enveloppe (6a), l'opercule étant maintenu de façon axialement mobile au moyen d'un support (6b) sur une ouverture de liaison dans un boîtier de soupape (5), qui, à l'autre extrémité, est muni d'une ouverture d'admission (6b) à peu près à l'opposé de l'opercule (6a), **caractérisé en ce que** l'opercule (6) est réalisé sous la forme de corps creux,
avec une ouverture tournée vers l'ouverture d'admission (7), pour former son espace creux, délimité par la surface d'enveloppe (6a) et de section transversale prédéterminée, et **en ce que** cet opercule (6) est susceptible de pivoter librement à une extrémité sur son support (6b), autour d'un axe d'articulation en tout cas imaginaire.

6. Soupape formant clapet selon la revendication 5, **caractérisé en ce que** l'ouverture présente la section transversale prédéterminée et ainsi est alignée avec la surface d'enveloppe (6a) de l'espace creux (5a).

7. Soupape formant clapet selon la revendication 5 ou 6, **caractérisé en ce qu'**au moins une partie de l'unité, formée de l'opercule (6) et du support (6b), est formée d'un matériau ayant une masse volumique inférieure à 1,034 g/cm³ de préférence inférieure à 1,028 g/cm³.

8. Soupape formant clapet selon la revendication 7, **caractérisé en ce qu'**au moins une partie de l'ensemble formé de l'opercule (6) et du support (6b), en particulier le support (6b), est formé en caoutchouc au silicone.
